Europäisches Patentamt

European Patent Office

Office européen des brevets

(11) Publication number: **0 406 485 A1**

# EUROPEAN PATENT APPLICATION

(21) Application number: 89201760.9

(22) Date of filing: 03.07.89

(51) Int. Cl.5: **A61M 1/36**

(43) Date of publication of application:
**09.01.91 Bulletin 91/02**

(84) Designated Contracting States:
**DE ES FR GB GR IT NL SE**

(71) Applicant: **NPBI Nederlands Produktielaboratorium voor Bloedtransfusieapparatuur en Infusievloeistoffen B.V.**
**Runde ZZ41**
**NL-7881 HM Emmer Compascuum(NL)**

(72) Inventor: **Feijen, Jan**
**Oude Grensweg 96**
**NL-7552 GD Hengelo(NL)**
Inventor: **Bruil, Anton**
**Olympiaplein 54**
**NL-7552 BM Hengelo(NL)**
Inventor: **Rejda, Theofil Wenzel**
**Noorddammerweg 42**
**NL-1187 CN Amstelveen(NL)**

(74) Representative: **Smulders, Theodorus A.H.J., Ir. et al**
**Vereenigde Octrooibureaux Nieuwe Parklaan 107**
**NL-2587 BP 's-Gravenhage(NL)**

(54) A method for the removal of leukocytes from a leukocyte-containing suspension and filter unit for use with the method.

(57) A method for removing leukocytes from a leukocyte-containing suspension by passing a leukocyte-containing suspension over a filter unit containing a filter comprising a continuous porous structure, and obtaining a leukocyte-poor liquid, whereby a continuous porous structure or a combination of porous structures is used having a pore gradient with decreasing pore size in the direction of the flow of the suspension.

# A METHOD FOR THE REMOVAL OF LEUKOCYTES FROM A LEUKOCYTE-CONTAINING SUSPENSION AND FILTER UNIT FOR USE WITH THE METHOD.

### State of the art

Blood transfusion, used for the treatment of diseases like leukemia and various chronic anemias, requires donor blood which has been freed of leukocytes (Sirchia et al., Vox Sang. 51 suppl. 1, 2-8, 1986). Leukocytes from donor blood may cause immunogenic reactions in the patient. These immunogenic reactions become more pronounced in patients regularly receiving blood transfusions, and cause phenomena like fever, headache, nausea, diarrhoea and throwing up. In some cases, alloimmunisation reactions can even cause death.

A variety of techniques has been developed for the removal of leukocytes from blood cell suspensions. Leukocyte-poor blood can be prepared by methods like centrifugation, washing, sedimentation with a density gradient, or succesively freezing and thawing the blood. All these techniques have the disadvantage that a low efficiency for separating the leukocytes is obtained. Besides this, the techniques are mostly time consuming, relatively expensive and inconvenient. To avoid these drawbacks, leukocyte filters are now applied. Using these filters, leukocytes can be easily and almost completely removed from blood cell suspensions at low costs.

The separation of leukocytes from blood by filtration over cotton wool was already described by Fleming in 1928 (Brit. J.Exp. Path. 60 , 281-286, 1928). Later Greenwalt et al. improved this method for application in blood transfusion (Transfusion, 2 , 221-229, 1962). In their method a column, filled with fibres of Nylon, is used. Application of leukocyte filters on a large scale, started only in the early 70's, stimulated by the results of clinical studies, carried out by Diepenhorst et al. (Vox Sang. 23 , 308-320, 1972).

After the introduction of the first commercially available leukocyte filter, consisting of a column filled with cotton wool, several modifications of this filter have been patented. In all cases, fibres are arranged in a so-called 'nonwoven structure', which allows depth filtration of the blood cell suspension. Improvement of the filter efficiency has been achieved by optimization of the non-woven structure and by selecting optimal size and density of the fibres, which play an important role in the distribution of filter pore sizes (US Pat. 4.701.267). Further improvements were achieved by application of filters made of other materials, like polyesters and polyamides (JP Pat. 55/135750).

Although the filters presently available have an acceptable capacity to remove leukocytes, their filtration efficiency may be further improved. Conventional filter structures, composed of packed fibres, have a broad pore size distribution, which is poorly defined. Using these fibres, it is almost impossible to created pores with the optimal size and a narrow size distribution.

### Description of the invention

Instead of porous structures obtained with fibres, one may obtain continuous porous matrices with well defined pore structures such as membranes or foams. Membranes or foams, composed of a continuous porous matrix, can be prepared by different techniques. Most of these techniques offer unique possibilities of adjusting pore structures. Considering this, membrane structures have important advantages over nonwoven structures.

It is a main object of the invention to provide a method for the removal of leukocytes from a leukocyte- containing suspension by passing said leukocyte-containing suspension through a filter unit allowing an improved filtration efficiency.

According to the invention there is provided a method for removing leukocytes from a leukocyte-containing suspension by passing the leukocyte-containing suspension through a filter unit containing a filter comprising a continuous porous structure and obtaining a leukocyte-poor liquid, said method being characterized in that a continuous porous structure or a combination of porous structures is used having a pore gradient with decreasing pore size in the direction of the flow of the suspension.

It has been reported that the filtration of leukocytes from blood can be carried out with polyester foams with an average pore size of 5-20$\mu$m (JP Pat. 55/136955). The recover of the leukocytes was 55%. The present invention, however, offers an improvement of these porous structures by application of pore gradients and by application of various polymeric materials which enhance the filtration efficiency. Until now such microporous systems with a continuous porous matrix having a pore gradient for the removal of leukocytes from a leukocyte-containing suspension such as blood, have not been described.

Initial experiments carried out with 'nonwoven' filter materials showed tht optimal leukocyte removal can be obtained when the average space between the polymer fibres approximates 8-10$\mu$m. This can be explained because leukocytes have a diameter of about 6 to 12$\mu$m. Red blood cells have a diameter of a approximately 8μm, but these cells are more flexible than leukocytes allowing an easy passage through pores of 6 - 12μm. This was substantiated by Lichtman, who showed that red blood cells may even pass capillaries with a diameter of 3μm while the transport of leukocytes through these capillaries is not possible. (J.Clin. Invest. 52 , 350-358, 1973). Based on these data membranes and foams were prepared with pore sizes in the order of about 10 μm.

Several methods may be used to obtain the desired materials. Two methods will be further discussed. These are a salt suspension technique and a thermally induced phase separation technique (TIPS).

The salt suspension technique comprises the use of a suspension of fine salt particles in a solution of polymer in an organic solvent (DE Pat. 3.525.731). After immersion of a thin film of this suspension, cast on a glass plate, in a suitable coagulation agent, the polymer precipitates. Salt particles may be washed out afterwards, leaving an open porous polymer matrix. By a proper choice of process conditions like concentration of salt and polymer,and size of the salt particles it is possible to adjust the pore size within certain limits.

The TIPS-technique is based on thermally induced phase separation processes in a polymer solution (US Pat.4.519.909). A polymer solution may separate in a polymer-rich and a polymer-poor phase, when the temperature is decreased to a critical point. Depending on the conditions , this leads to a dispersion of one phase in the other.After a further decrease of temperature the microphase structure may be fixed by freezing. When the solid solvent is removed by extraction or freeze drying, an open porous polymer matrix remains. Optimization of the pore size distribution is possible by adjustment of process conditions like cooling rate and polymer concentration.

Compared with the salt suspension technique, the TIPS-procedure is most convenient. A great flexibility in the use of different polymers is an important advantage over the the salt suspension technique. Besides this, the TIPS-method is not restricted to the preparation of thin membranes. Also foams with a thickness of several centimeters, may be prepared. Using TIPS it is even possible to obtain pore size gradients, by the application of temperature gradients over the filter. (Caneba and Soong, Macromolecules 18 , 2538-2545. 1985).

Within the context of the invention the term "continuous porous structure" as is used herein and in the claims relates to membranes as well as to foam materials in the form of a film or a sheet having a thickness of less than 500 μm, for membranes and at least 500 μm for foams respectively.

Although the invention is described mainly with regard to the filtration of whole blood, it is not limited thereto but generally relates to the removal of leukocytes from leukocytes-containing suspensions. For example, the invention is also applicable to blood concentrates.

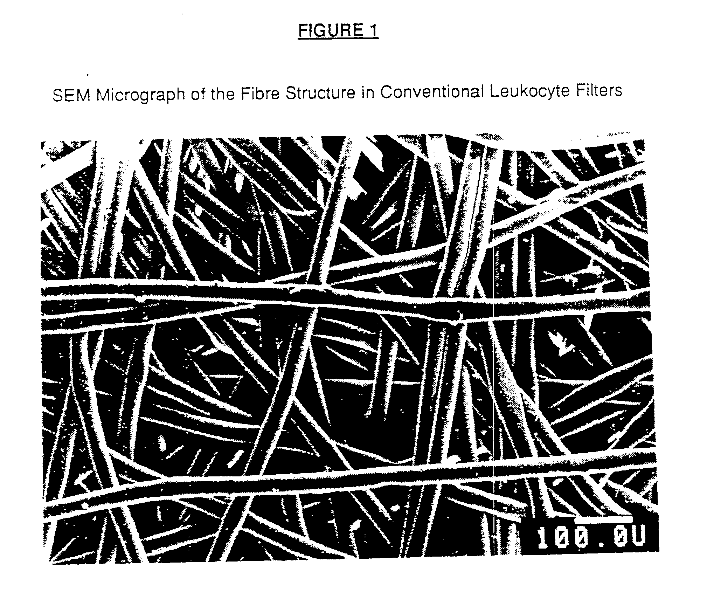

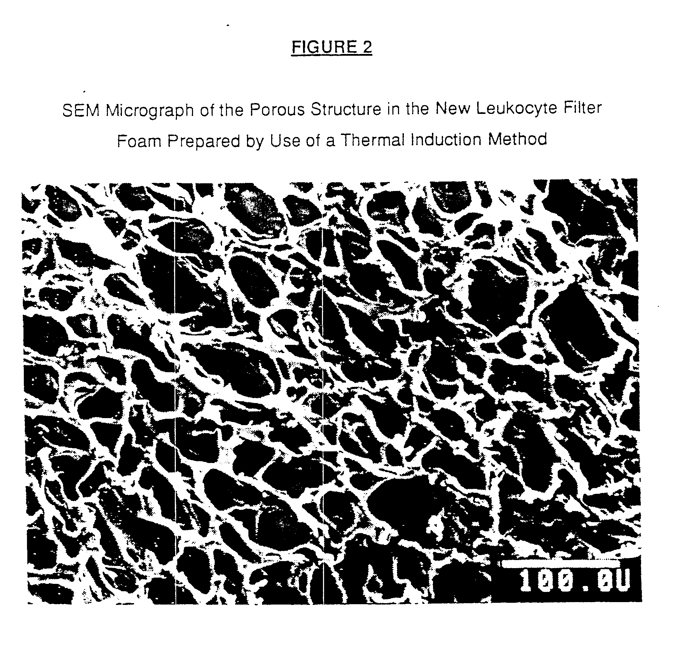

Using the salt suspension technique, polyurethane membranes with different pore size distributions were prepared. Using the TIPS-technique membranes with comparable pore size distributions were prepared, composed of different materials like polyurethane, polystyrene and cellulose acetate. Also polyurethane foams with a thickness of about 5 mm were prepared. The structural difference between the new filter materials according to the invention and conventional filter materials is clearly illustrated by figures 1 and 2.

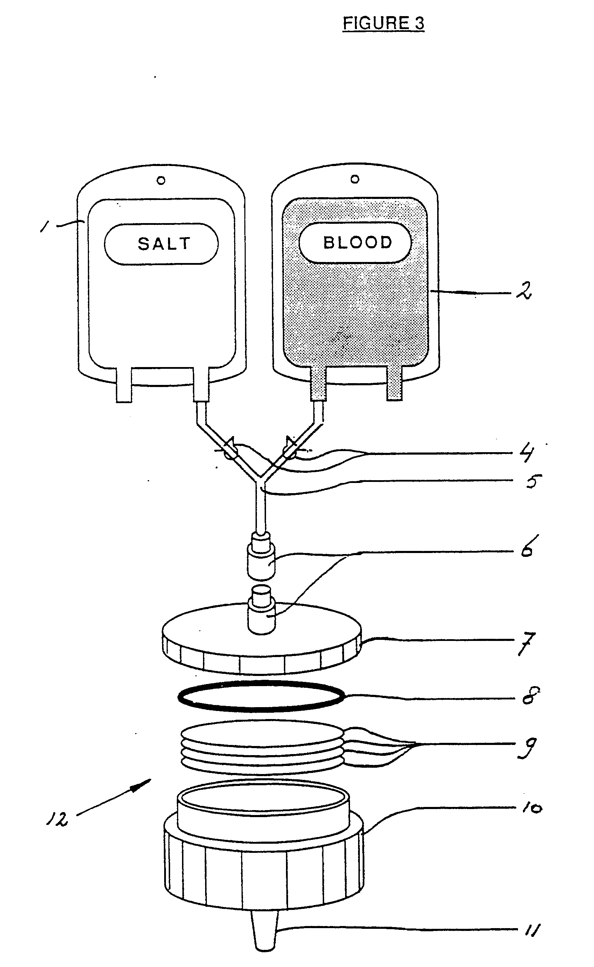

Leukocyte filters were prepared by stacking porous membranes. Filters were also prepared using single foams or combinations of foams with membranes. With regard to the filtration of blood, membranes and/or foams 9 were placed in a polycarbonate holder 10 of a filter unit 12 as is illustrated in figure 3.A silicon rubber ring 8 is inserted to prevent leaking. The filter 9 is first rinsed with physiological salt solution which is contained in reservoir 1, before filtration. Reservoir 2 contains the blood which is to be filtrated. The filter holder 10 is provided with a distribution cap7 by which it may be closed via screw thread means at its top side.

The reservoirs 1 and 2 are connectable to the filter unit 12 via T-junction 5 which itself is coupled to the distribution cap 7 by means of coupling means 6.

The supply of either the salt solution or the blood is regulated with closing clips 3 and 4, respectively.

The filtrate as leukocyte-poor blood leaves the filter unit 12 at the outlet 11. Filtrations are performed by application of hydrostatic pressure over the filter, which pressure doesnot vary more than 10% during the experiment.

In a typical filter experiment the filtration characteristics of polyurethane filters with an equal pore size distribution also called herein symmetrical filters, which filters fall outside the invention, and filters with a gradient in pore size also called asymmetrical filters which filters belong to the invention, are compared. Using fresh citrated blood containing no microaggregates, both the leukocyte retention and the blood flow under constant pressure were measured as a function of time. There was also measured the total volume of blood which could be filtrated before the filter got clogged. With the new filters it was possible to filtrate considerable amounts of leukocytes from whole blood. No hemolysis of the red blood cells was measured. It was found that membranes with pore sizes about

5-10μm retained leukocytes most efficiently but these filters also got clogged very soon. Once most of the pores are clogged by retained leukocytes, the blood flow rapidly decreases. Surprisingly a composed membrane filter with decreasing pore size in the flow direction, showed comparable leukocyte retentions as symmetrical or homogeneous filters without a pore size gradient while through the former filters higher flow rates of the blood could be achieved. Moreover the gradient filter die not show any plugging effects during the last stage of the filtration. It will be shown more in detail in the following examples that a leukocyte filter with a gradient in pore size, shows a larger capacity for leukocyte removal than a leukocyte filter without a gradient.

Also filters, prepared from different materials , were compared. For example, filters composed of polyurethane, polystyrene and cellulose acetate, showed very different filtration characteristics. The polyurethane filter showed a much higher efficiency than the polystyrene filter. The cellulose acetate filter showed even better results. Without being limited to any theoretical explanation this large material influence may be explained by the fact that the adhesion of leukocytes on various materials differs depending on the material surface free energy (Absolom et al., Trans. Am. Soc. Artif. Intern. Organs 25 , 152-158, 1979).

However, also other polymers which are known for their suitability for use in contact with blood such as human blood may be used for preparing the filters according to the invention. These polymers comprise polyester, polyamide, polyether, cellulose or other cellulose derivates, polyacrylate, polymethacrylate, polyacrylonitril, polyvinylalcohol, polyvinylchloride, polypropylene, polyethylene, fluorinated polyolefins, polysulphone, silicon rubbers and blends of said polymers or copolymers derived thereof such as poly(ethylene-vinylacetate).

Considering the excellent properties of the tested structures, it is believed that the new filter materials according to the invention have a very good potential for medical application in the field of leukocyte filtration.

Example 1

Application of a salt suspension method

Different polyurethane membranes were prepared by application of the salt suspension method. By choosing the size of the salt particles, the average pore size could be adjusted between about 10-40μm.

In a typical experiment, polyurethane membranes with a pore size distribution of about 8-20μm were prepared. Sodium citrate was sieved over a 400 mesh sieve to a fraction with granular diameters less than 38μm. These particles, in 13-14 fold weight excess, were suspended in a solution of 7% w/v polyetherurethane (Pellethane ® 2363-80A) in dimethylformamide. The degassed suspension was then case to a thin film on a flat glass surface. After this the system was immersed in a coagulation bath of ethanol, to precipitate the polymer. Finally the salt could be removed from the membrane by extraction with water.

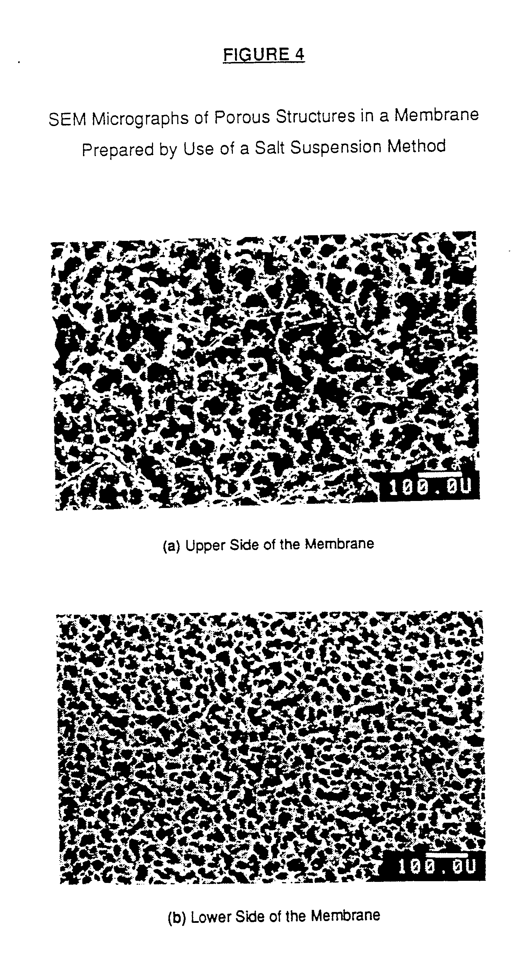

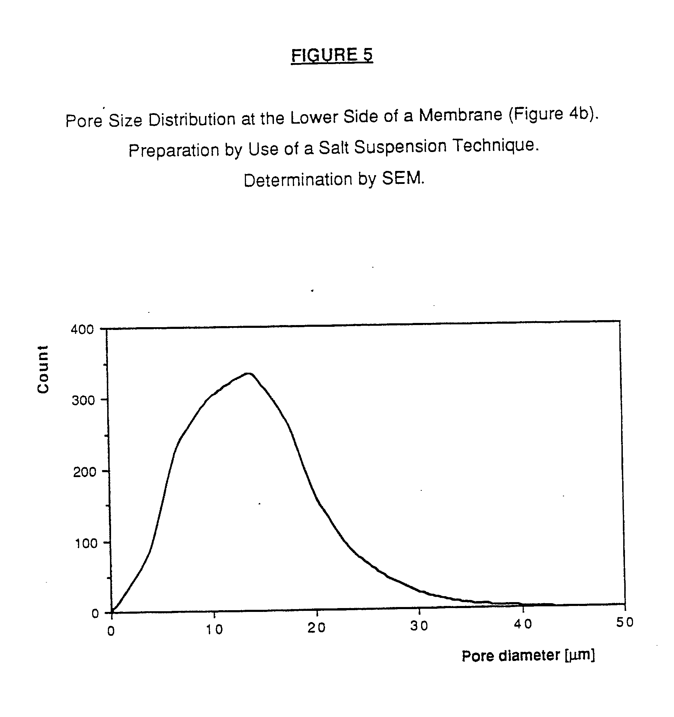

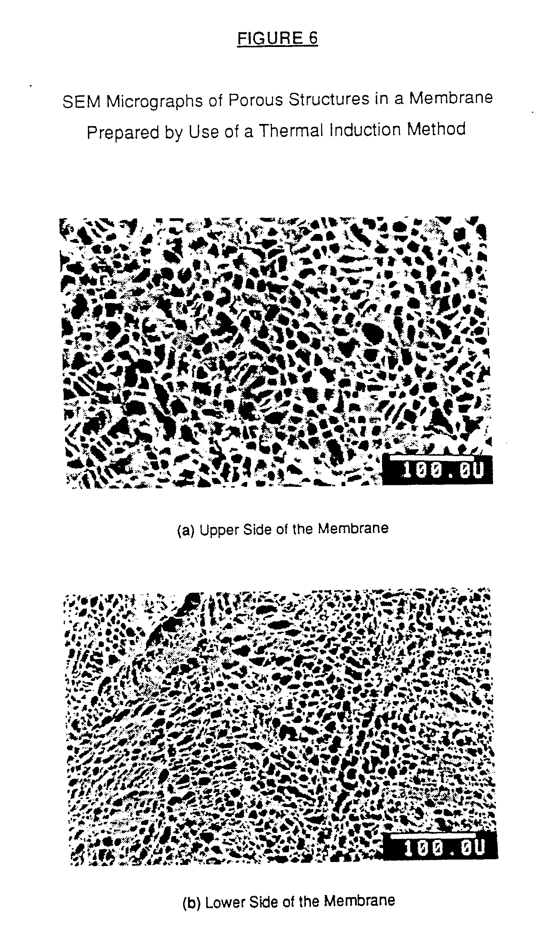

The pore size distribution in the membrane was studied by 'scanning electron microscopy' (SEM), see figure 4. From SEM it followed that the microporous structure of the membrane is open, cellular and almost symmetrical. Figure 5 shows the pore size distribution curve of the lower side of the membrane. An average pore size of 14±6μm was measured from this curve. The membrane thickness was about 250μm.

Example 2

Application of a thermal induction method

a) Preparation of membranes

Polyurethane membranes with a small pore size distribution of about 5μm were prepared by application of the TIPS method. A degassed solution of 5% w/v polyurethane (Pellethane ® 2363-80A) in dry dioxane was cast to a thin film on a flat glass surface and quickly cooled down at -20°C. A porous membrane resulted after extraction of the frozen dioxane in an ice/water bath.

The pore size distribution in the membrane was studied by 'scanning electron microcopy' (SEM), see figure 6. From SEM it followed that the microscopic structure of the membrane is open, cellular and almost symmetrical. An average pore size of 6±3μm at the lower side of the membrane and a membrane thickness of about 150μm were measured.

With the same method also other materials than polyurethane can be used. For example, polystyrene and cellulose acetate membranes were prepared under the conditions already described for polyurethane. The membranes showed comparable microstructures with the polyurethane membranes.

b) Preparation of foams

An open polyurethane foam with a thickness of about 5 mm was prepared by application of the TIPS method. A degassed solution of 10% w/v polyetherurethane (Pellethane ®2363-75D) in dimethylsulfoxide was extracted from the polymer in an ice/water bath.

The pore size distribution in the membrane was studied by 'scanning electron microscopy' (SEM), see figure 2. From SEM it followed that the microporous structure of the membrane is open, cellular and almost symmetrical. The pore sizes varied between 20-80$\mu$m.

Example 3

Filtration of leukocytes

a)Filtration over 'symmetric' composed polyurethane filters

Test filtrations were performed with different poly urethane membranes, prepared according to the descriptions in examples 1 and 2a. Membranes with average pore sizes of 6-, 14-, 17-, 21- and 43$\mu$m were used. Filters with a total thickness of about 2 mm were cut from a stack of 8 membranes with a similar structure. The filters were then placed in the polycarbonate holder 10, shows in figure 3, and rinsed with 100 ml salt solution.

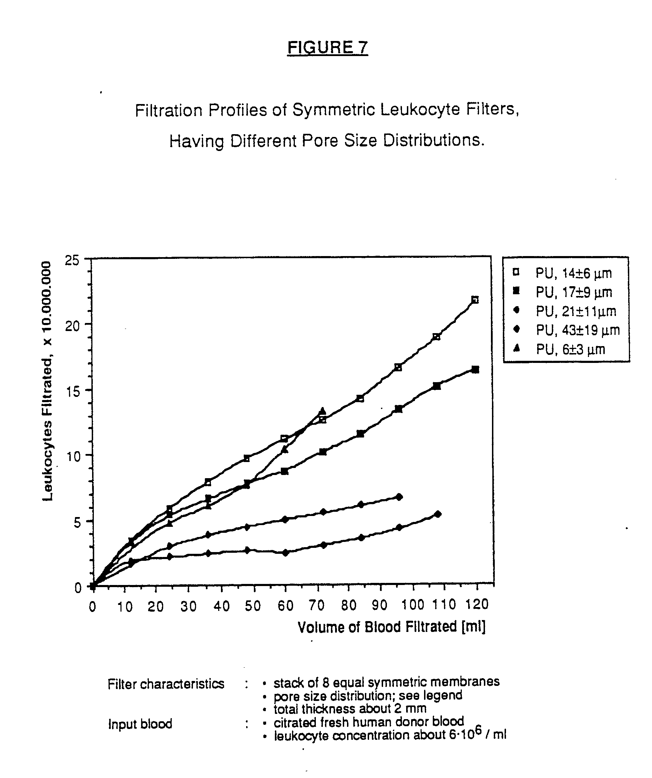

The filtration was performed with fresh citrated donor blood, containing about $6.10^6$ leukocytes/ml. In each case more than 100 ml blood could be filtrated before the filter got clogged. Filters with average pore sizes of 21- and 43$\mu$m did not get clogged at all. The filtration efficiency was studied by analysing the eluted blood with a Coulter ® counter. It was found that a considerable amount of leukocytes was removed from the blood throughout the entire course of the filtration. It was also shown that the number of removed leukocytes was dependent on the average pore size, as illustrated in figure 7. Almost no loss or hemolysis of the red blood cells was observed.

It was concluded that filters with an average pore size of about 6-14$\mu$m have the highest leukocyte removal efficiency.

b) Filtration over 'symmetric' filters of different materials.

Structural comparable membranes were prepared from polyetherurethane (Pellethane ® 2363-80A), polystyrene and cellulose acetate by application of the TIPS technique described in example 3a.

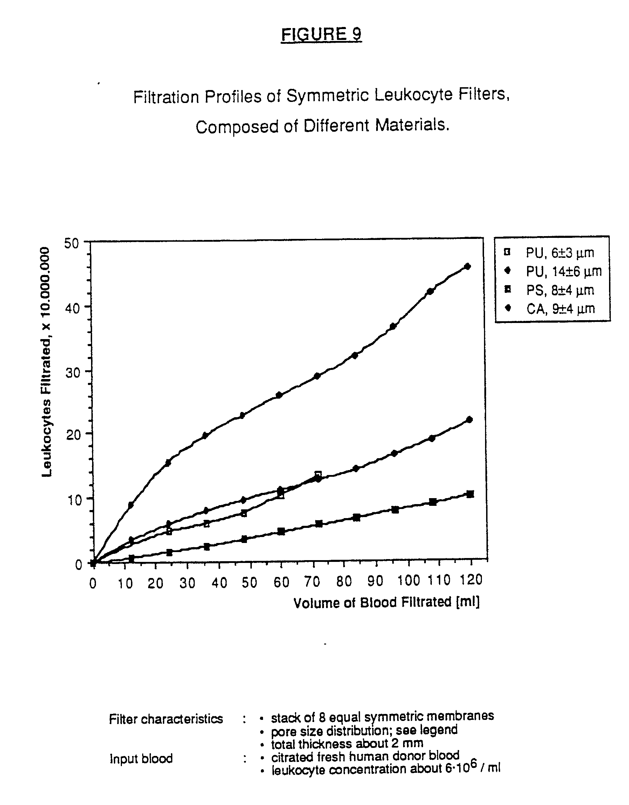

Due to structural similarities, no significant differences in blood flow were measured. In figure 9 it is illustrated that the leukocyte removal efficiency of the cellulose acetate filter was found to be 2 respectively 4 times higher than the polyurethane and polystyrene filter.

It was concluded that the filtermaterial plays an important role in the improvement of filtration efficiency.

c) Filtration over an 'asymmetric' composed polyurethane filter

An 'asymmetric' or 'gradient' filter, in which the pore size decreases in the blood flow direction, was obtained by using different membranes. Membranes also used in example 3a, were ordered in the sequence 14-, 17-, 21-, and 43$\mu$m. Each membrane was used two times so that the filter consists of 8 membranes in total. The filter was then placed in the polycarbonate holder 10, shown in figure 3, and rinsed with 100 ml salt solution.

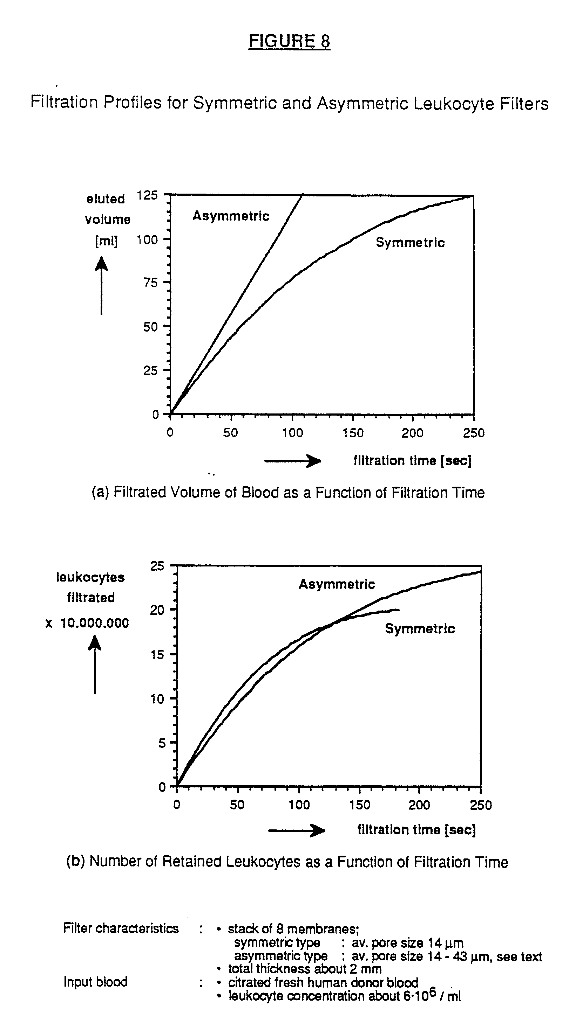

A test filtration was performed comparable to that of example 3a. Both the blood flow and the leukocyte removal were measured and compared to the results of a similar experiment with a symmetric membrane with an average pore size of 14$\mu$m. The results are shown in figures 8a and 8b. Compared with the symmetric filter, blood flow was higher at the same hydrostatic pressure. Moreover this filter did not get clogged at the end of the filtration, as illustrated by figure 8a. Following from figure 8b, the leukocyte retaining capacity was not altered appreciably.

It is concluded that, due to a more efficient depth filtration, gradient filters show a larger leukocyte removal capacity than symmetric filters.

In addition to the above it will be clear that according to the invention a continuous porous structure also comprises a structure obtained by stacking different membranes. Also between the membranes a spacer means may be applied.

## Claims

1. A method for removing leukocytes from a leukocyte-containing suspension by passing a leukocyte-containing suspension over a filter unit containing a filter comprising a continuous porous structure, and obtaining a leukocyte-poor liquid, characterized in that a continuous porous structure or a combination of porous structures is used having a pore gradient with decreasing pore size in the direction of the flow of the suspension.

2. A method according to claim 1, characterized in that the continuous porous structure is a membrane or a combination of two or more stacked membranes, said membranes each having a thickness of less than 500µm.

3. A method according to claim 1, characterized in that the continuous porous structure is a foam material or a combination of two or more stacked foam materials, said foam materials each having a thickness of at least 500µm.

4. A method according to claims 1-3, characterized in that the continuous porous structure is a stacked combination of one or more membranes and one or more foam materials.

5. A method according to claims 1-4 characterized in that a continuous porous structure is used having a pore gradient with decreasing pore size varying from an average pore size of 10-200 µm to 3-20 µm in the direction of the flow of the leukocyte-containing suspension.

6. A method according to claim 5, characterized in that the average pore size varies from 10-50 µm to 3-15 µm in the direction of the flow of the leukocyte-containing suspension.

7. A method according to claims 1-6, characterized in that the continuous porous structure is made from a polymer, a metal, a ceramic, glass, a composite or a combination of two or more of said materials.

8. A method according to claim 7, characterized in that the continuous porous structure is made from a polymer or a combination of polymers.

9. A method according to claim 8, characterized in that the continuous porous structure is made from polyurethane, poly ester, polyamide, polyether, cellulose, cellulose acetate or other cellulose derivate, polyacrylate, polymethacrylate, polyacrylonitril, polyvinylalcohol, polyvinylchloride, polypropylene, polyethylene, fluorinated polyolefin, polystyrene, polysulphone, silicon rubbers and blends of said polymers or copolymers derived thereof such as poly(ethylene-vinylacetate).

10. A method according to claim 9, characterized in that the polymer is polyurethane,polystyrene or cellulose acetate.

11. A method according to claim 10, characterized in that cellulose acetate is used.

12. A filter unit for use with the method according to claims 1-11 for removing leukocytes from a leukocyte containing suspension comprising a container provided with at least one inlet conduit means and at least one outlet conduit means, the container having a filter contained therein, characterized in that said filter is a continuous porous structure or a combination of porous structures having a pore gradient with decreasing pore size in the direction of the flow of the leukocyte-containing suspension.

13. A filter unit according to claim 12, characterized in that the continuous porous structure is a membrane or a combination of two or more stacked membranes, said membranes each having a thickness of less than 500µm.

14. A filter unit according to claim 12, characterized in that the continuous porous structure is a foam material or a combination of two or more stacked foam materials each having a thickness of at least 500µm.

15. A filter unit according to claims 12-14, characterized in that the continuous porous structure is a stacked combination of one or more membranes and one or more foam materials.

16. A filter unit according to claims 12-15, characterized in that the continuous porous structure has a pore gradient with decreasing pore size varying from an average pore size of 10-200 µm to 3-20 µm in the direction of the flow of the leukocyte-containing suspension.

17. A filter unit according to claim 16, characterized in that the average pore size varies from 10-50 µm to 3-15 µm in the direction of the flow of the leukocyte-containing suspension.

18. A filter unit according to claims 12-17, characterized in that the continuous porous structure is made from a polymer, a metal, a ceramic, glass, a composite, or a combination of two or more of said materials.

19. A filter unit according to claim 18, characterized in that the continuous structure is made from a polymer.

20. A filter unit according to claim 19, characterized in that the continuous porous structure is made from polyurethane, polyester, polyamide, polyether, cellulose, cellulose acetate or other cellulose derivative, poyacrylate, polymethacrylate, polyacrylonitril, polyvinylalcohol, polyvinylchloride, polypropylene, polyethylene, fluorinated polyolefin, polystyrene, polysulphone, silicon rubbers and blends of said polymers or copolymers derived thereof such as poly(ethylene-vinylacetate).

21. A filter unit according to claim 20,characterized in that the polymer is polyurethane, polystyrene or cellulose acetate.

22. A filter unit according to claim 21, characterized in that the polymer is cellulose acetate.

## FIGURE 1

SEM Micrograph of the Fibre Structure in Conventional Leukocyte Filters

100.0U

## FIGURE 2

SEM Micrograph of the Porous Structure in the New Leukocyte Filter

Foam Prepared by Use of a Thermal Induction Method

## FIGURE 3

## FIGURE 4

SEM Micrographs of Porous Structures in a Membrane

Prepared by Use of a Salt Suspension Method

(a) Upper Side of the Membrane

(b) Lower Side of the Membrane

## FIGURE 5

Pore Size Distribution at the Lower Side of a Membrane (Figure 4b).
Preparation by Use of a Salt Suspension Technique.
Determination by SEM.

## FIGURE 6

SEM Micrographs of Porous Structures in a Membrane
Prepared by Use of a Thermal Induction Method

(a) Upper Side of the Membrane

(b) Lower Side of the Membrane

## FIGURE 7

Filtration Profiles of Symmetric Leukocyte Filters,

Having Different Pore Size Distributions.

Filter characteristics  :  • stack of 8 equal symmetric membranes
                            • pore size distribution; see legend
                            • total thickness about 2 mm
Input blood             :  • citrated fresh human donor blood
                            • leukocyte concentration about $6 \cdot 10^6$ / ml

## FIGURE 8

Filtration Profiles for Symmetric and Asymmetric Leukocyte Filters

(a) Filtrated Volume of Blood as a Function of Filtration Time

(b) Number of Retained Leukocytes as a Function of Filtration Time

Filter characteristics    :   • stack of 8 membranes;
                                     symmetric type    : av. pore size 14 μm
                                     asymmetric type   : av. pore size 14 - 43 μm, see text
                                 • total thickness about 2 mm
Input blood                  :   • citrated fresh human donor blood
                                 • leukocyte concentration about $6 \cdot 10^6$ / ml

14

## FIGURE 9

Filtration Profiles of Symmetric Leukocyte Filters,

Composed of Different Materials.

Filter characteristics   :   • stack of 8 equal symmetric membranes
                              • pore size distribution; see legend
                              • total thickness about 2 mm
Input blood              :   • citrated fresh human donor blood
                              • leukocyte concentration about $6 \cdot 10^6$ / ml

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 313 348 (PALL CORP.)<br>* Page 7, lines 5-27; page 11, lines 13-23; page 22, lines 29,55-59; figure 43; table 13 * | 1,2,5-13,16-22 | A 61 M  1/36 |
| A | US-A-4 704 203 (C.C. REE)<br>* Column 6, lines 18-56 * | 1,3,4,9,10,18,19,20,21 | |
| A | EP-A-0 024 601 (BIOTEST-SERUM-INSTITUT)<br>* Page 5, lines 1-16; page 6, paragraph 4 * | 1,2 | |
| A | EP-A-0 190 020 (SHILEY INC.)<br>* Page 9, paragraph 2 * | 1 | |

TECHNICAL FIELDS SEARCHED (Int. Cl.5)

A 61 M

The present search report has been drawn up for all claims

| Place of search | Date of completion of the search | Examiner |
|---|---|---|
| THE HAGUE | 12-10-1989 | KERRES P.M.G. |